# EUROPEAN PATENT SPECIFICATION

(11) **EP 2 298 380 B1**
(45) Date of publication and mention of the grant of the patent: **25.04.2012**
(21) Application number: 09170805.7
(22) Date of filing: 21.09.2009
(51) Int. Cl.: A61M 1/36

(54) **A blood reservoir incorporating a vapor trap**
Blutreservoir mit integrierter Dampffalle
Réservoir de sang incorporant un piège à vapeur

(43) Date of publication of application: 23.03.2011
(73) Proprietor: Tamari, Yehuda, Oyster Bay, NY 11771 (US)
(72) Inventor: Tamari, Yehuda, Oyster Bay, NY 11771 (US)
(74) Representative: Körber, Martin Hans

(56) References cited:
- US-A- 5 931 646
- US-A- 6 017 493
- US-A1- 2007 110 612

## Description

### BACKGROUND OF THE INVENTION

### FIELD OF THE INVENTION

The invention comprises a venous reservoir with or without a cardiotomy reservoir that also incorporates a vapor trap. The combined units provide a self contained venous reservoir ready for vacuum assisted venous drainage (VAVD) without the VAVD kit needed for prior art devices. The combination simplifies and reduces setup time.

### DESCRIPTION OF THE PRIOR ART

During cardiopulmonary bypass, blood flow from the venous side of the patient to a venous reservoir depends on the resistance of the fluid conduit between patient and reservoir and the pressure difference between patient and reservoir. Typical prior art venous cardiotomy reservoirs are represented by Fini's US Patent 6,287,270 (Fini) and Thor's US Patent 5,411,705 (Thor). When the venous reservoir is maintained at atmospheric pressure, that pressure difference is the height difference between patient and reservoir; the resulting flow is referred to as gravity drainage. Venous drainage by gravity alone provides inadequate return during procedures such as minimally invasive cardiac surgery and bypass via femoral cannulation. Usually it is the resistance of the venous cannula that limits the flow rate. Vacuum assisted venous drainage (VAVD) is a technique that overcomes flow limitations by applying suction to the venous reservoir thereby increasing the pressure difference between the venous cannulation site and venous reservoir. VAVD allows for a decrease in the inner diameter (ID) of the venous line, thereby reducing prime volume and enabling the use of a smaller internal diameter cannula, which translates to an easier insertion, better surgical view and a smaller surgical incision.

When vacuum is applied to the venous reservoir to assist in pulling blood out of the patient then water vapor evaporating off the blood (e.g. at 37°C) condenses on the cooler walls of the tubing between the vacuum source and the venous reservoir and can drip back into the blood. Water condensate dripping back into the blood can compromise sterility and damage the blood. Current systems eliminate that problem by inserting a sterile vapor trap between the vacuum source and the venous reservoir, a step that increases setup time and cost.

US 2007/0110612 Al suggests using a liquid reserving chamber 15 to collect water vapor condensate as part of a defoaming device that includes a body portion having an internal space for blood to flow and a foam reserving chamber. The se two chambers are connected via a connecting pipe.

US 6,017,493A describes vacuum assisted venous drainage (VAVD) as a useful clinical tool to improve clinical outcome by using vacuum to enhance venous blood flowing from the patient to a venous reservoir.

The present invention incorporates a vapor trap with the venous or cardiotomy reservoir eliminating the need for a standalone vapor trap.

### BRIEF SUMMARY OF THE INVENTION

The present invention as defined in claim 1 comprises a blood reservoir that uses vacuum to enhance blood flow from the patient that incorporates a separate chamber designed to trap condensing water vapor associated with the use of vacuum. The blood reservoir and the vapor trap chamber are in fluid communication along their top assuring that vacuum applied to the top of the vapor trap chamber is equally applied to the blood reservoir.

An objective of the present invention is provide a vapor trap in the vacuum side of a blood reservoir, said trap replacing a standalone vapor trap thereby reducing cost and setup time. Further advantageous features are defined in the dependent claims.

### BRIEF DESCRIPTION OF THE DRAWINGS

FIG. 1 is a line drawing of the front view of a prior art venous cardiotomy reservoir with a standalone vapor trap placed between air purging port and a vacuum source.
FIG. 2 is a line drawing of the front view of one embodiment of the present invention illustrating a vapor trap formed as a separate chamber combined with a venous cardiotomy reservoir of FIG. 1.
FIG. 3 is a line drawing of the front view of another embodiment of the present invention illustrating a vapor trap incorporated within a venous cardiotomy reservoir.

### DETAILED DESCRIPTION OF THE INVENTION

Reference should now be made to the drawings wherein the same reference numerals are used throughout to designate the same or similar parts.

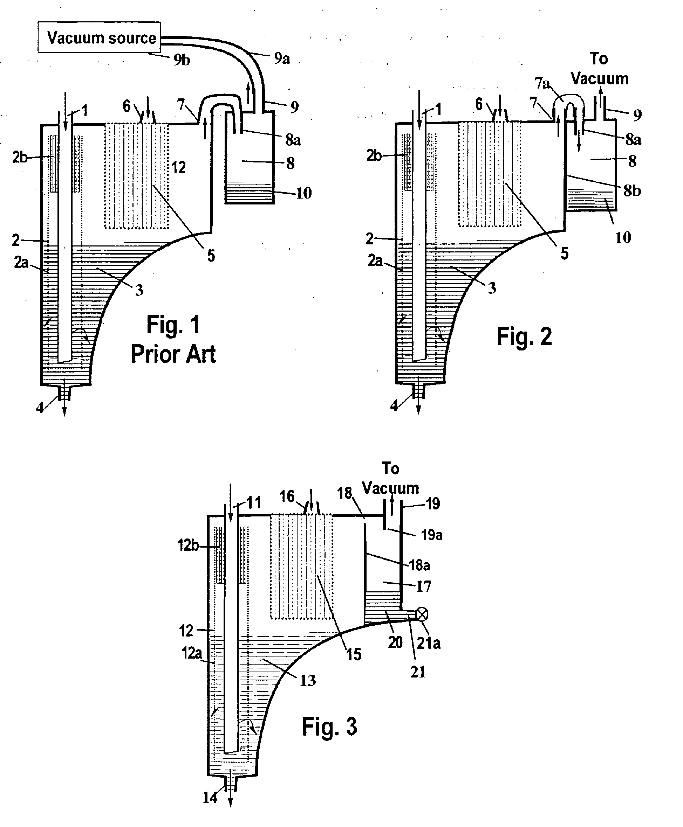

FIG. 1 is a schematic representation of a typical prior art venous cardiotomy reservoir similar to that described in Nogawa's US Patent 5,931,646 (Nogawa). Briefly, venous blood enters inlet 1 located along the top of chamber 3 is directed downward into inlet blood chamber 2 having screened wall 2a. Blood flows from inlet blood chamber 2 across screen 2a and into outlet blood chamber 3. Air bubbles entering inlet blood chamber 2 are inhibited by screen 2a from crossing into outlet blood chamber 3 and any foam formed by the air in the blood is broken up by defoamer 2b. The air and foam free blood in outlet blood chamber 3 exits via outlet port 4 located along the bottom of chamber 3. Blood being sucked from the surgical field, or from being diverted from the purge line of the top of an arterial filter, is directed into cardiotomy filter 5 via cardiotomy inlet port 6 located along the top of chamber 3. Air that enters outlet blood chamber 3, either from air being purged from the blood in inlet blood chamber 2, or from air being purged by the cardiotomy filter, rises to the top of outlet blood chamber 3 and is then purged via air purge port 7 located along the top of chamber 3. When VAVD is desired, then sterile vapor trap 8 is inserted between vacuum source 9b and air purging port 7 and vacuum is applied via tubing 9a to outlet port 9 located at the top of vapor trap chamber 8. The water vapor that condensates along tube 9a between vacuum source 9b and outlet 9 of the vapor trap chamber 8, is collected at the bottom of vapor trap chamber 8 and is identified as 10. Preferably the fluid communication between outlet port 9 of vapor trap 8 and the top of outlet blood chamber 3 and the top of inlet blood chamber 2 allows vacuum applied to vapor trap 8 to equal the vacuum in outlet blood chamber 3 and inlet blood chamber 2.

FIG. 2 is a schematic representation of an embodiment of the present invention wherein a vapor trap is incorporated into the venous cardiotomy blood reservoir shown in Fig. 1. Here vapor trap chamber 8 shares wall 8b and, can also share a top, with outlet blood chamber 3 and is in fluid communication with chamber 3 via interconnecting tube 7a. This embodiment duplicates the function of the standalone vapor trap shown in Fig. 1 while reducing manufacturing costs by reducing the number of components needed as well as the time needed by the end user to setup.

FIG. 3 is a schematic representation of another embodiment of the present invention wherein the vapor trap is incorporated into the venous cardiotomy blood reservoir shown in Fig. 1. Briefly, venous blood enters inlet 11 located along the top of chamber 13 and is directed downward into inlet chamber 12 having screened wall 12a. Blood flows from inlet chamber 12 across screen 12a and into outlet chamber 13. Air bubbles entering inlet chamber 12 are inhibited by screen 12a from crossing into outlet chamber 13 and any foam formed by the air in the blood is broken up by defoamer 12b. The air and foam free blood in outlet chamber 13 exits via outlet port 14 located along the bottom of chamber 13. Blood being sucked from a surgical field, or from being diverted from the purge line of an arterial filter, is directed into cardiotomy filter 15 via cardiotomy inlet port 16 located along the top of chamber 13. A second chamber housed within outlet chamber 13 forms vapor trap chamber 17. Vapor trap 17 is in fluid communication with the top of outlet chamber 13 via channel 18 allowing vacuum applied to air purge port 19 of vapor trap chamber 17 to be equally applied to the top of outlet chamber 13 and to the top of inlet chamber 12.

Fig. 3 also illustrates two safety features that can be incorporated into vapor trap chamber 17. First, means are incorporated to allow the end user to empty water 20 from vapor trap chamber 17. Here valve 21a is added to the bottom of chamber 17 that, when opened, allows water 20 to be removed from chamber 17. Alternatively, valve 21 a can be replaced by clampable tube 21 that the user can open/close using a tubing clamp.

The second safety feature is a structure that prevents condensate water 20 from overflowing into outlet blood chamber 13 via fluid channel 18. This is achieved by having bottom 19a of air purge port 19 extend below channel 18. Thus, should water 20 rise in vapor trap chamber 17, it would reach bottom 19a of outlet port 19 and be sucked out by the vacuum source, thereby preventing water 20 from reaching fluid channel 18 and avoiding water 20 from overflowing into outlet blood chamber 13 of the reservoir.

The vapor trap chamber can be formed by injection molding as a part of either the inlet blood chamber or the outlet blood chambers of the venous reservoir or as part of the cardiotomy. For example, a vapor trap chamber of this invention can be integrated into enclosed inlet chamber 8 or enclosed outlet chamber 9 of combined cardiotomy and venous blood reservoir 1 of Thor's venous reservoir shown in his FIG. 1. This can be achieved by retooling the molds forming Thor's reservoir. Similarly, a vapor trap chamber of this invention can be integrated into either venous reservoir 3 or cardiotomy reservoir 4 of Fini's combined venous blood reservoir and a cardiotomy reservoir shown in his FIG. 1. And in yet another example, a vapor trap can be incorporated into any one of chambers 3, 10a, 10b or 26 shown in Fig. 4a, 4b and 4c in Tamari's pending EPO patent application number 05 826 776.6-2320 and published as WO 2006/057650. For each of the aforementioned examples, the vapor trap is structured to assure that accumulated water condensate cannot overflow into the blood chamber sharing a wall with the vapor trap.

The structures of Fig. 1, 2, and 3 can be manufactured by means well known in the art in the field of medical devices and as exemplified by aforementioned Nogawa's, Fini's and Thor's reservoirs.

It should be understood that the venous blood reservoir can be used for gravity drainage by simply not applying vacuum to air purging port 19 of vapor trap chamber 17. This embodiment, however, does not form part of the present invention.

It should be further understood that the blood reservoir can be venous reservoir, a cardiotomy reservoir, of as described above, a venous and cardiotomy reservoir combination.

Finally it should be understood that a comprehensive description of each of the applications of the invention is beyond the scope of a patent application and therefore the aforementioned descriptions are given as illustrations and should not be used to limit the scope of the invention, as defined by the appended claims.

## Claims

1. A combination of a blood reservoir used to pull blood from a patient using vacuum and a vapor trap, said combination comprising:
a) a blood chamber (3) having an inlet port and an outlet port; and
b) a vapor trap comprising:
(i) a chamber (8) to collect water condensate;
(ii) a fluid communication (7a) with the blood chamber;
(iii) at least one wall (8b) that is shared with the blood chamber;
(iv) an air purging port (9) designed to accept a vacuum source; and
(v) a structure (7a, 8a, 9; 19a, 19) that precludes water vapor condensate associated with the use of vacuum from reaching the blood chamber;
wherein vacuum applied to the air purging port of the vapor trap is equally applied to the blood chamber.

2. The combination of a blood reservoir used to pull blood from a patient as claimed in Claim 1, wherein the blood chamber is a venous reservoir.

3. The combination of a blood reservoir used to pull blood from a patient as claimed in Claim 1, wherein the blood chamber is a cardiotomy reservoir.

4. The combination of a blood reservoir used to pull blood from a patient as claimed in Claim 1, wherein the blood chamber is a venous cardiotomy reservoir.

5. The combination of a blood reservoir used to pull blood from a patient as claimed in one of the Claims 1 through 4 further comprising means (21a, 21) that allow the end user to empty water condensate from the vapor trap chamber.

6. The combination of a blood reservoir used to pull blood from a patient as claimed in Claim 5 wherein the means to allow the end user to empty water condensate from the chamber of the vapor trap is a valve (21a).

7. The combination of a blood reservoir used to pull blood from a patient as claimed in Claim 5 wherein the means to allow the end user to empty water condensate from the chamber of the vapor trap is a clampable tube (21) that the user can open/close using a tubing clamp.

## Patentansprüche

1. Kombination aus Blutreservoir zur Blutentnahme eines Patienten mittels Vakuum und Dampfsperre aufweisend:
a) eine Blutkammer (3) mit Einlaßöffnung und Auslassöffnung; und
b) eine Dampfsperre bestehend aus:
(i) einer Kammer (8) zum Sammeln kondensierten Wassers;
(ii) einer Fluidverbindung (7a) mit der Blutkammer;
(iii) wenigstens einer die Blutkammer teilende Wand (8b);
(iv) einer Luftreinigungsöffnung (9) zur Verbindung mit einem Vakuumerzeuger; und
(v) eine Struktur (7a, 8a, 9, 19a, 19) die bei Vakuum die Wasserdampfkondensation in der Blutkammer ausschließt;
wobei Vakuum sowohl an die Luftreinigungsöffnung der Dampfsperre als auch an die Blutkammer angelegt wird.

2. Kombination nach Anspruch 1, wobei
die Blutkammer ein venöses Reservoir darstellt.

3. Kombination nach Anspruch 1, wobei
die Blutkammer ein Kardiotomiereservoir darstellt.

4. Kombination nach Anspruch 1, wobei
die Blutkammer ein venöses Kardiotomiereservoir darstellt.

5. Kombination nach einem der Ansprüche 1 bis 4,
ferner Mittel (21 a, 21) aufweisend, die es dem Endanwender erlauben, in der Dampfsperre kondensiertes Wasser zu entfernen.

6. Kombination nach Anspruch 5, wobei
die Mittel zum Entfernen in der Dampfsperre kondensierten Wassers ein Ventil (21a) darstellen.

7. Kombination nach Anspruch 5, wobei
die Mittel zum Entfernen in der Dampfsperre kondensierten Wassers einen abklemmbaren Schlauch (21) darstellen, welchen der Anwender mittels einer Schlauchklemme öffnen und schließen kann.

## Revendications

1. Combinaison d'un réservoir de sang utilisé pour prélever du sang d'un patient en utilisant un vide et un piège à vapeur, ladite combinaison comprenant:
a) une chambre de sang (3) ayant un orifice d'entrée et un orifice de sortie; et
b) un piège à vapeur comprenant:
(i) une chambre (18) pour recueillir le condensat d'eau;
(ii) une communication fluidique (7a) avec la chambre de sang;
(iii) au moins une paroi (8b) partagée avec la chambre de sang;
(iv) un orifice de purge d'air (9) conçu pour accepter une source de vide; et
(v) une structure (7a, 8a, 9; 19a, 19) qui empêche que le condensat de vapeur d'eau associé à l'utilisation du vide atteigne la chambre de sang;
où le vide appliqué à l'orifice de purge d'air du piège à vapeur est également appliqué à la chambre de sang.

2. Combinaison d'un réservoir de sang utilisé pour prélever du sang d'un patient selon la revendication 1, où la chambre de sang est un réservoir veineux.

3. Combinaison d'un réservoir de sang utilisé pour prélever du sang d'un patient selon la revendication 1, où la chambre de sang est un réservoir de cardiotomie.

4. Combinaison d'un réservoir de sang utilisé pour prélever du sang d'un patient selon la revendication 1, où la chambre de sang est un réservoir de cardiotomie veineuse.

5. Combinaison d'un réservoir de sang utilisé pour prélever du sang d'un patient selon l'une quelconque des revendications 1 à 4, comprenant en outre des moyens (21a, 21) qui permettent à l'utilisateur final de vider le condensat d'eau de la chambre à piège à vapeur.

6. Combinaison d'un réservoir de sang utilisé pour prélever du sang d'un patient selon la revendication 5, où des moyens pour permettre à l'utilisateur final de vider le condensat d'eau de la chambre du piège à vapeur est une vanne (21a).

7. Combinaison d'un réservoir de sang utilisé pour prélever du sang d'un patient selon la revendication 5, où les moyens pour permettre à l'utilisateur final de vider le condensat d'eau de la chambre du piège à vapeur est un tube apte à être serré que l'utilisateur peut ouvrir/fermer en utilisant une pince de tube.
